Europäisches Patentamt

European Patent Office

Office européen des brevets

(11) Publication number: **0 207 392**
**B1**

(12) **EUROPEAN PATENT SPECIFICATION**

(45) Date of publication of patent specification: 27.12.90

(51) Int. Cl.⁵: **G 01 N 33/52** // G01N33/84, G01N31/22

(21) Application number: **86108403.6**

(22) Date of filing: **20.06.86**

(54) Multilayer ion test means.

(30) Priority: **02.07.85 US 751185**
**02.07.85 US 751257**

(43) Date of publication of application:
**07.01.87 Bulletin 87/02**

(45) Publication of the grant of the patent:
**27.12.90 Bulletin 90/52**

(84) Designated Contracting States:
**AT BE CH DE FR GB IT LI LU NL SE**

(56) References cited:
**EP-A-0 033 707**
**EP-A-0 125 554**
**EP-A-0 125 555**
**GB-A-2 065 302**
**US-A-4 042 335**
**US-A-4 166 093**

**CLINICAL CHEMISTRY, vol. 28, no. 9, September
1982, pages 1857-1861, Washington, US; S.C.
CHARLTON et al.: "Solid-phase determination of
potassium"**

(73) Proprietor: **MILES INC.**
**1127 Myrtle Street**
**Elkhart Indiana 46514 (US)**

(72) Inventor: **Charlton, Steven C.**
**1533 Strong Avenue**
**Elkhart, IN 46514 (US)**
Inventor: **Fleming, Roger L.**
**3107 May Street**
**Niles, MI 49120 (US)**
Inventor: **Hemmes, Paul**
**1825 Brockwood Drive**
**Elkhart, IN 46514 (US)**
Inventor: **Lau, Arthur L.Y.**
**1038 Bancroft Circle no. F**
**Mishawaka, IN 46544 (US)**

(74) Representative: **Dänner, Klaus, Dr. et al
c/o Bayer AG Konzernverwaltung RP
Patentabteilung
D-5090 Leverkusen 1 Bayerwerk (DE)**

**Description**

1. Introduction

The present invention relates to the measurement of ions, in particular ions in aqueous solution, and to a multilayer test means or devices for performing such measurements. The invention provides a convenient format for determining the presence and/or concentration of ions whereby results are available shortly after contact with an aqueous test sample. Cumbersome, expensive electronic equipment, such as ion-specific electrodes, flame photometers, atomic absorption spectrophotometers or the like, is not needed.

A multilayer format for ion testing can be used advantageously to measure ion content of highly colored fluids. For example, the multilayer format is particularly useful for whole blood measurements. The multilayer format is unique in that it can be used to provide information on ultrafilterable or "free" calcium and to determine serum potassium levels using a whole blood sample without interference due to lysed blood cells.

Although it is the potassium ion content of serum which provides a critical clinical indication of many conditions leading to muscle irritability and excitatory changes in myocardial function, among others, it would be convenient to use a whole blood specimen and eliminate the separation of blood cells from the serum prior to measurement. However, blood cells contain large amounts of potassium ion and if lysed during the measurement, would lead to large errors in the values reported. Therefore, a whole blood device capable of measuring serum potassium which is independent of "hematocrit" or cell content of the blood would provide a convenience not available with present potassium ion tests.

2. Background of the invention

Methods for determining ions in solution include flame photometry, atomic absorption photometry and ion-specific electrodes. Test strip formats have been disclosed in EP—A—0 125 554, EP—A—0 125 555, EP—A—0 153 641 and EP—B—0 041,175 assigned commonly herein. Preferred reporter substances have been disclosed in U.S. Patent No. 4,540,520 and 4,552,697 commonly assigned herein.

Multilayer formats for the determination of analytes by chemical reaction of the analyte with the components in the reagent layer have been disclosed (see for example U.S. Patent No. 3,992,158 to Przybylowicz et al). Kitajima et al. in U.S. Patent No. 4,356,149 discloses a multilayer integral element for the chemical analysis of blood wherein the reagent layer is composed of a hydrophilic binder and fine hydrophobic particles dispersed in that binder, which particles contain the reagent capable of producing a color change with the component being analyzed. U.S. Patent No. 4,356,149 to Kitajima et al. is directed toward eliminating extra layers made necessary by incompatible reagent components. Kitajima deals only with the determination of a neutral analyte with a reaction requiring the separation of incompatible reagent components. U.S. Patent No. 4,255,384, to Kitajima et al. concerns a multilayer integrated element for the chemical analysis of blood.

Further in Clin. Chem. *28/9*, pages 1857—1861 (1982) a solid phase colorimetric determination of potassium is described which is based on a nonpolar organic film.

U.S. Patent No. 4,166,093 is directed to a reduction of detectable species migration in elements for the analysis of liquids.

3. Brief description of the drawings

Figure 1 diagrammatically depicts a multilayer device format particularly suited for whole blood determination. Fig. 1a depicts a supported three layer device; Fig. 1b depicts a supported two layered device. The figures are described in detail in Section 7 entitled "Multilayer Test Device".

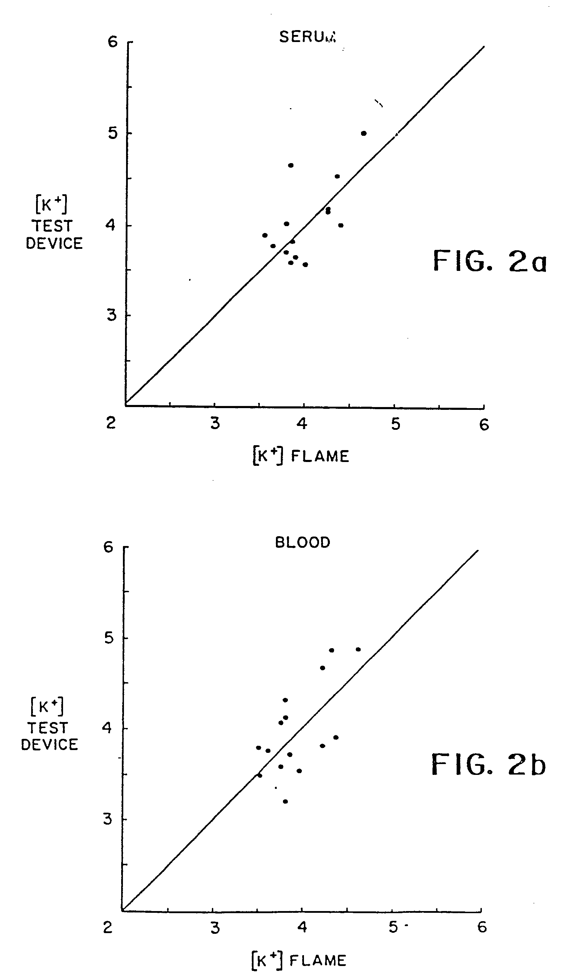

Figure 2 shows data for a multilayer device given in the comparison Example 8.1. The graph depicts apparent millimolar potassium ion concentration [K$^+$] determined with a comparative test device compared to millimolar [K$^+$] obtained with the reference flame photometry method. Figure 2a shows serum data and Figure 2b shows whole blood data.

Figure 3 shows the decrease of hematocrit dependence of a whole blood potassium determination when sodium chloride is incorporated into the multilayer test device. The graph depicts data obtained as shown in Example 8.2.

4. Summary of the invention

This invention resides in the discovery of a multilayer test means for detecting the presence of an ion in an aqueous test sample and determining its concentration. The multilayer device is preferred for determining the concentration of cations such as K$^+$, Na$^+$, Ca$^+$, Mg$^{++}$ and Li$^+$ in whole blood samples. The multilayer device includes at least a reagent layer and a reflecting layer, and optionally also includes an opacifying layer and a transparent support member.

The reagent layer of the multilayer test means comprises *either* a hydrophilic carrier matrix incorporated with finely divided globules of a hydrophobic vehicle which globules contain two principal ingredients: an ionophore capable of forming a complex with a specific ion to be determined and a reporter substance capable of interacting with the complex of the ionophore and the ion to produce a detectable response such as a change in, or the appearance of, color or fluorescence; *or* a substantially non-polar, nonporous carrier matrix incorporated with an ionophore and reporter having the characteristics described above. For the determination of cations a neutral reporter, having a dissociable proton which proton is capable of dissociating upon interaction of the reporter with the complex of the ionophore and the cation to produce a detectable response, is preferred.

The addition of the reflecting layer and optionally the opacifying layer with a nonporous, nonpolar reagent layer, permit the addition of such components as a buffer and an interferant removal substance. The addition of the buffer allows ion determination with an unbuffered, undiluted sample. An interferant removal substance can complex with an interfering ion, permitting greater selectivity and precision.

The multilayer test means for whole blood potassium comprises sufficient sodium chloride which is incorporated in a layer of the multilayer device to provide hematocrit independence.

The multilayer format is preferred for whole blood determinations, as electrolyte concentration can be determined colorimetrically without washing or wiping the device.

### 5. Definitions

The following definitions are provided to clarify the scope of the present invention and to enable its formulation and use.

5.1 The term "ionophore" includes molecules capable of selectively forming a complex with a particular ion in a nonpolar environment to the substantial exclusion of others. For example the cyclic polyether 2,3 - naphtho - 1,4,7,10,13 - pentaoxacyclopentadeca - 2 - ene (sometimes known as 2,3 - naphtho - 15 - crown - 5 and called Potassium Ionophore I herein) binds to potassium ions selectively to form a cationic complex. Included in the term are coronands, such as are crown ethers, cryptands, podands and antibiotic type ionophores such as valinomycin and macrotetralide actins.

5.2 A "reporter substance" is one which is capable of interacting with an ionophore/ion complex to produce a change in, or appearance of, color or other detectable response. Preferred reporters are neutral compounds having a dissociable proton, which is capable of dissociating upon interaction of the reporter with the ionophore/ion complex to produce a detectable response. With the loss of a proton, the reporter becomes charged, effecting a change in electron distribution. The change in electron distribution produces a detectable response. The expression "reporter substance" includes phenolic compounds, such as p-nitrophenol, and naphthols which are relatively colorless in the nonionized state, but which color upon ionization, and fluorescent compounds which produce more or less fluorescence upon a change in electron distribution. The reporter substance can also be one which can trigger a detectable response together with other components. For example, the change in electron distribution in the reporter substance, caused by interaction with the complex, can in turn facilitate the interaction of the reporter with another component which would then produce a detectable response. Preferred reporters are disclosed in U.S. Patent Nos. 4,540,520 and 4,552,697.

5.3 By "interacting" is meant any coaction between a reporter substance and an ionophore/ion complex which leads to a detectable response. The interaction between an ionophore/cation complex and preferred reporters having a dissociable proton will cause the reporter to lose a proton thus producing a detectable response. An example of the reporter substance interacting with the complex is the case where the reporter is changed by the complex from a colorless to a colored state, such as in the case of p-nitrophenol.

5.4 The expression "detectable response" is used herein as a change in, or occurrence of, a parameter in a test means system which is capable of being perceived, either by direct observation or instrumentally, and which is a function of the presence of a specific ion in an aqueous test sample. Some detectable responses are the change in, or appearance, of, color, fluorescence, reflectance, pH, chemiluminescence and infrared spectra.

5.5 The term "globule" as used herein refers to spherical or quasi-spherical globes, balls or other shaped particles of a substance such as form in biphasic suspensions or emulsions. When an oil-in-water suspension or emulsion is formed, the oil (hydrophobic) phase exists as spherical entitities surrounded by a more or less continuous aqueous phase. The more energy supplied in forming the suspension, the smaller the size of the globules. Likewise, the globular size can be controlled by additives such as detergents and other emulsifying agents.

Also included in the meaning of the term "globule" are finely divided particles of a solid material. Thus, where the hydrophobic vehicle is a hydrophobic solid material such as a polymer, it can be ground or otherwise finely divided into solid particles.

5.6 By "hydrophilic" is meant that characteristic of a substance to have a strong or pronounced tendency to bind or absorb water. Included in the term are those materials which undergo swelling or formation of reversible gels with water, or which are wettable or permeable by water, or which form aqueous solutions.

5.7 As used herein, "substantially nonpolar" is intended as meaning that quality of a substance not to exhibit a substantial dipole moment or electrical polarity. In particular, it includes nonionic substances and substances which are dielectric. A "substantially nonpolar" material is intended to mean a hydrophobic material. Hydrophobic generally means that the material is immiscible with water. With preferred reporters used herein, hydrophobic is additionally taken to mean that in the absence of the complex of ionophore

3

and ion in the nonpolar (or hydrophobic) layer, the reporter substance is in the protonated form.

5.8 The term "nonporous" is intended to mean substantially impervious to the flow of water. Thus, a nonporous carrier matrix is one which precludes the passage of water through it, one side to the other. For example, a polyvinyl chloride film will be considered for the purposes herein as being nonporous.

5.9 The phase "hydrophobic vehicle" as used herein means a substance or substances which are capable of isolating the ionophore and reporter from the aqueous phase formed by contact of the sample with the test device. Thus the vehicle can be a solid or a liquid or a combination thereof, provided that it increases the ability of the ionophore/ion complex and the reporter to coexist in the hydrophobic globule. Useful vehicles include high boiling liquids such as nitrophenyloctylether, sometimes called "plasticizers" and polymers such as polyvinylchloride.

## 6. Multilayer test means

A multilayer test means of the present invention is composed of a reagent layer containing the ionophore and reporter substance incorporated in a carrier matrix and at least a reflecting layer, in laminar relationship. The reagent layer and suitable carrier matrices have been fully described in EP—A—0 125 554 and EP—A—0 125 555. EP—A—0 125 555 describes a nonporous, nonpolar carrier matrix containing an ionophore capable of interacting with the ion of interest and a suitable reporter substance. The nonporous nonpolar carrier is referred to herein as the "film" format.

EP—A—0 125 554 describes a reagent layer in the form of an emulsion wherein finely divided hydrophobic globules containing the ionophore and reporter are incorporated in a hydrophilic carrier matrix respectively. This type of reagent layer is referred to herein as the "emulsion" format. For the determination of cations, preferred reporters are neutral compounds having a dissociable proton, which proton is capable of dissociating upon interaction of the reporter with the complex of the ionophore and the cation to produce a detectable response. The preferred detectable response is color. Multilayer test devices will usually be read by reflectance measurement taken from the side opposite the side of sample application through a transparent support member.

Additional components can be added to the reagent layer. Light scattering centers, described more fully below, can be added to either reagent layer format. When the reagent layer is a hydrophilic carrier containing finely divided hydrophobic globules, the hydrophilic carrier provides a matrix for the incorporation of buffers, sodium chloride and preferred water soluble interferant removal substances. However, the addition of the reflecting layer and optionally the opacifying layer, provides a vehicle for the addition of sodium chloride and buffer which might otherwise destabilize the reagent layer. When the film format is used, the additional layers are needed to allow the incorporation of these components into the test device.

## 6.1 Light scattering centers

When instrumental reading by reflectance is used, it is advantageous to incorporate light scattering centers with reagent layer. The use of such centers effectively increases the precision of the assay by reducing the effect of variations in dry film thickness. Light scattering centers can be produced by incorporating insoluble, inorganic particles such as titanium dioxide particles or equivalents such as barium sulfate, calcium carbonate, magnesium oxide, aluminum oxide, zinc oxide, lead oxide, microcrystalline cellulose or talc. A working range for incorporation of titanium dioxide particles is up to about 40% weight percent of a coating emulsion; a preferred range is from 0.5 to 15 weight percent of coating emulsion for the reagent layer. A particle size of less than one µm is preferred.

## 6.2 Sodium chloride addition to decrease hematocrit dependence

If a hydrophilic carrier incorporated with hydrophobic globules is used as the reagent layer of a multilayer test means for a whole blood electrolyte determination, sodium chloride is incorporated with the hydrophilic carrier. The addition of approximately 0.1 to 0.2 M sodium chloride has been found to be sufficient to obviate the hematocrit dependence otherwise seen with whole blood potassium tests. The addition of salt is particularly advantageous in the multilayer format preferred for whole blood determinations and will be discussed more fully later in the specification.

## 6.3 Interferant removal substance

Body fluids normally contain many cations, such as sodium ion ($Na^+$), potassium ion ($K^+$), calcium ion ($Ca^{++}$) and magnesium ion ($Mg^{++}$). Although the ionophore will usually be chosen for its selectivity for the desired analyte-ion, in some cases the presence of other cations could interfere with the coaction of the ionophore with the desired analyte-ion and decrease the accuracy of the test. For example, Sodium Ionophore I will bind sodium in preference to calcium ion in a ratio of approximately 4 to 1. In samples where the ratio of sodium ion to calcium ion is less than 4 to 1, it may be necessary to prevent the interaction of calcium ion with the ionophore to ensure the proper relationship between sodium ion concentration and the detectable response. An interferant removal substance can be provided to obviate this problem.

An interferant removal substance can be incorporated into the hydrophilic matrix or into the hydrophobic globules. In a preferred embodiment, the removal substance is designed to interact with an interfering cation so as to keep it in the aqueous phase formed by contact with the aqueous fluid sample, or otherwise prevent cation interaction with the ionophore in the hydrophobic globule. For example, ethylenediamine tetraacetic acid (EDTA) and ethyleneglycol *bis*(aminoethyl)tetraacetic acid (EGTA) are water soluble compounds which form complexes with divalent cations, such as calcium ion. If EDTA is incorporated with the hydrophilic carrier matrix of the emulsion reagent layer for the determination of sodium ion, EDTA will preferentially bind calcium ion in an aqueous sample containing sodium and calcium ions. The bound calcium ion will not substantially interfere with the formation of the ionophore/ sodium ion complex in the hydrophobic globule. In addition, ionophores can be used to remove interfering cations if they are specific for the interfering ion and are water soluble or are modified chemically to increase their water solubility without decreasing their ability to interact with the interferant. For example, Sodium Ionophore III (chemical name given in abbreviation list, structure can be found in EP—A—0 153 641 can be modified by the addition of solubilizing groups, such as ($-SO_3H$) groups, to the benzene rings to increase its water solubility without decreasing its ability to interact with sodium ion. Other compounds such as *trans* - 1.2 - diaminocyclohexane - N,N,N',N' - tetraacetic acid can also be used advantageously.

## 6.4 Buffer

A buffer or combination of buffers can be incorporated with the hydrophobic carrier matrix of the emulsion reagent layer. Upon contact with an aqueous fluid sample, the buffer redissolves into the aqueous phase thus created, raising or lowering the pH to the desired level for the generation of the detectable response to proceed. With preferred reporters capable of losing a dissociable proton upon interaction with the ionophore/ion complex, the buffer mains a suitable pH for the reaction to proceed.

Suitable buffers include, but are not limited to, bis[2 - hydroxyethyl]imino - tris[hydroxymethyl] - methane; 1,3 - bis[tris(hydroxymethyl)methylamino] - propane, N,N - bis - (2 - hydroxyethyl)glycine, tris(hydroxymethyl)amino - methane, N - [2 - acetamido] - 2 - iminodiacetic acid; N - 2 - hydroxyethylpiperazine - N',3 - propanesulfonic acid; 3[N - tris(hydroxymethyl) - methylamino - 2 - hydroxypropanesulfonic acid; tetramethylammonium borate; 3 - (cyclohexylamino)propane sulfonic acid and tetramethylammonium phosphate.

The preferred pH range depends on the reporter substance. Therefore, the choice of the buffer is determined by the reporter substance used and to some extent by the desired detectable response. For example, when 7-decyl MEDPIN is used as the reporter, the preferred pH range is from 6 to 8.5. However, when a reporter substance having a higher pKa for the dissociable proton is used, a higher pH range will be preferred. Similarly when a reporter having a lower pKa for the dissociable proton is used, a lower pH range will be preferred. When the detectable response is a color change, the buffer can influence the degree of such detectable response, and a particular buffer can be chosen for color intensity optimization. For example, the useful pH range for the reporter, 7-decyl MEDPIN, occurs from pH 6 to 8.5 where the color change is from orange to blue. A higher pH, pH 8.5 to 10, gives shades of dark blue which are difficult to distinguish visually, and a lower pH, pH 5 to 6, gives shades of pale yellow, also difficult to distinguish visually. Both pH extremes could be used with instrumental analysis, although the best instrumental precision occurs at the pH range of from about 6 to 8.5. Determination of a suitable pH is a routine laboratory experiment.

## 7. Multilayer test means

A multilayer test means for the determination of an ion in an aqueous fluid sample can be prepared by the addition of a reflecting layer and optionally an opacifying layer on top of the reagent layer.

The reflecting layer contains a material or mixture of materials in the form of insoluble inorganic particles which provide a background to aid the user in determining the detectable response in the reagent layer of the device. In a preferred embodiment for whole blood determinations, the purpose of the reflecting layer is to screen the color of the red blood cells in the sample being tested from the color change to be observed by the user. A preferred substance for "screening purposes" is titanium dioxide. However, other materials can be used, for example, barium sulfate, calcium carbonate, aluminum oxide, magnesium oxide, zinc oxide, lead oxide, talc and microcrystalline cellulose. Such material is contained in the reflecting layer in an amount of from 5 to 40 weight percent, preferably 15 to 40 weight percent, based on the total weight of the reflecting layer. Such materials generally have a particle size of less than one μm. The materials generally have a dry coating weight of 2.5 to 75 g/m², (grams per square meters), preferably 10 to 25 g/m².

In addition to the inorganic substances, the reflecting layer can contain a hydrophilic substance such as gelatin. The hydrophilic substance is contained in an amount of 2 to 8 weight percent, preferably 2.5 to 5.5 weight percent, based on the total weight of the reflecting layer. Suitable hydrophilic substances include gelatin, agarose, poly(vinyl alcohol), poly(propyleneimine), copolymers of acrylic acid, carrageenan and alginic acid. Commonly, the remainder of the coating solution for the reflecting layer is water.

Additionally, the reflecting layer may contain one or more wetting agents (surfactants) and/or one or more suspending agents. An example of a wetting agent which can be employed in the reflecting layer is Triton® X-100, a polyoxyethylene surfactant available from Sigma Chemical Co. For example, in a test involving the determination of potassium, 0 to 0.5 weight percent Triton® X-100 can be utilized.

Examples of suspending agents for use in the reflecting layer include gelatin, alginate and hydrophilic urethanes.

The reflecting layer has a dry coating weight in the range of 2.5 to 75 g/m² (grams per square meters), preferably 10 to 25 g/m². To prepare a multilayer test means, the reflecting layer is coated on the dried reagent layer and dried at about 40°C for about 10 minutes.

In a preferred embodiment, an opacifying layer is coated on top of the reflecting layer, i.e., the opacifying layer is optional. The opacifying layer has a wet layer thickness (thickness when applied) of 10 to 50 μm preferably 15 to 35 μm. The opacifying layer contains substantially inert particles for imparting an opaque appearance to the layer suspended in a hydrophilic substance. Hydrophilic substance and suspending agents used can be those as described in the reflecting layer. Such particles can be, for example, carbon black particles.

The additional layers in the multilayer device can optionally include a buffer, an interferant removal substance and/or sodium chloride as described previously for the reagent layer. Pre-buffering allows the test means or multilayer device to be used with unbuffered and undiluted sera or whole blood. Although buffer can be added to the hydrophilic matrix with the emulsion reagent layer, the presence of additional layers provides a vehicle for more buffer capacity which might otherwise have an adverse affect on the emulsion if incorporated directly into the reagent layer.

A multilayer device including an opacifying layer is prepared by coating the opacifying layer on top of the reflecting layer and drying the whole device again at about 40°C for about 10 minutes.

In using the multilayer device, a sample is applied on top of the uppermost layer, i.e., the reflecting layer if no opacifying layer is employed, or the opacifying layer; and the detectable response, usually color, or the reagent layer after a certain timed delay is read from below. The multilayer format can be coated on or affixed to a transparent support. Useful transparent supports include polystyrene, polyester, polycarbonate, cellulose acetate and polyethyleneterephthalate. Polyethyleneterephthalate or cellulose acetate subbed to accept gelatin are preferred.

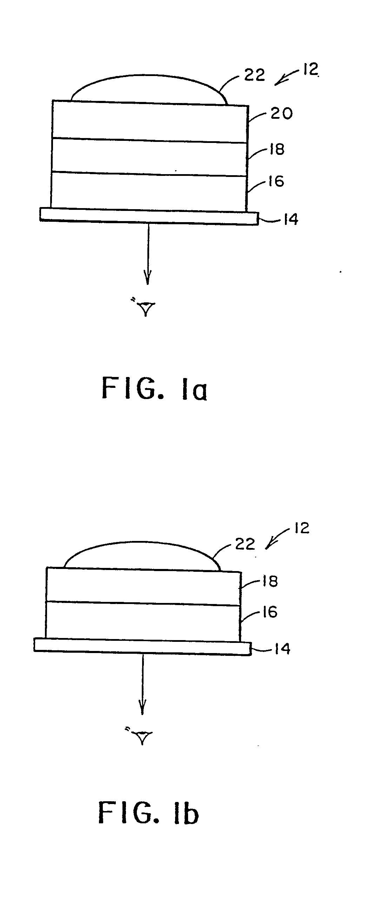

A multilayered device 12 in accordance with a preferred embodiment of the present invention is depicted in Figures 1a and 1b. As shown in Figure 1b the multilayered device 12 is composed of a transparent support layer 14 on top of which is coated a reagent layer 16. A reflecting layer 18 is coated on top of the reagent layer 16. As shown in Figure 1a an opacifying layer 20 can optionally be disposed on top of reflecting layer 18. A sample, 22, is placed on top of opacifying layer 20. Color development can be read from the bottom of layer 14 by the human eye or using a instrument such as a SERALYZER® reflectance photometer (Ames Division, Miles Laboratories, Inc.) which has been adapted for ion determinations.

The presence of the added reflecting layer or reflecting and opacifying layers effectively seal the reagent layer from the outside environment. Accordingly, the multilayer device protects the reagents in that without the added reflecting layer or added reflecting and opacifying layers the reagents would be exposed and, therefore, susceptible to damage due to mechanical contact. The presence of the added reflecting layer or added reflecting and opacifying layer also serve to prolong the stability of generated color.

The use of the reflecting layer, or reflecting and opacifying layers, also provides a blocking mechanism to cells, proteins and other macromolecules, thus permitting the use of whole blood without washing or wiping the test device. In addition, due to the filtration capabilities of these layers, a multilayer test device for calcium measures ultrafilterable calcium, as bound calcium should not reach the reagent layer. Single layer and multilayer calcium test device could be used to provide information on total calcium versus ultrafilterable calcium in a test sample.

The working and preferred concentration ranges for a multilayer test means responsive to potassium ion are given below. Preferred ranges are given for the determination of serum potassium by reflectance of an Ames SERALYZER® reflectance photometer. These concentrations can be used as a starting point for the determination of useful concentration ranges for the determination of other ions with other ionophores and reporters. The concentrations of ionophore and reporter refer to concentration in the volume of organic solvent used, other concentrations are defined as grams per 100 grams of solution. The reagent layer thickness for the multilayer test device can be from 25 μm to 500 μm, with a preferred wet thickness being from 150 μm to 200 μm.

| | Working | Preferred |
|---|---|---|
| I. Reagent Layer | | |
| Film thickness | 25—500 μm | 150—200 μm |
| Ionophore | 10—500 mM | 80—160 mM |
| Reporter | 10—120 mM | 30—60 mM |
| Light scattering | | |
| centers (optional) | 0—40% | 0.5—15% |

6

# EP 0 207 392 B1

If the reagent layer is a hydrophilic carrier containing hydrophobic globules:

| | | |
|---|---|---|
| Hydrophobic vehicle | 5—15% | 7—12% |
| Hydrophilic carrier | 5—15% | 7—12% |
| Buffer (optional) | 0—0.5 M | 0.2—0.5 M |
| Interferant removal substance (optional) | 0—30 g/L | 10—20 g/L |
| Wetting substance (optional) | 0—3 g/L | 1—2 g/L |
| Sodium chloride | 0—0.6 M | 0.5—0.15 M |

If the reagent layer is a nonporous, nonpolar carrier matrix

| | | |
|---|---|---|
| Polymer | 5—25% w/w | 5—25% w/w |
| Hydrophobic vehicle | 8—50% w/w | 8—50% w/w |

II. Optional Multilayers
   1. Reflecting layer

| | | |
|---|---|---|
| Titanium dioxide | 5—40% | 15—40% |
| Gelatin | 1—6% | 2—6% |
| Sodium chloride | 0—0.6 M | 0.05—0.5 M |
| Dry coating weight | 2.5—75 g/m² | 10—25 g/m² |

   2. Opacifying Layer

| | | |
|---|---|---|
| Carbon black | 3—30% | 7—15% |
| Gelatin | 1.0—12% | 1.5—6% |
| Sodium chloride | 0—1 M | 0—0.6 M |
| Film thickness (wet) | 10—50 μm | 15—35 μm |

8. Examples

Abbreviations used in the examples are as follows:

Square brackets, [ ], are used to designate ion concentration in millimoles per liter (mM) in the linear regression equations. All percent concentrations are given in weight per deciliter unless otherwise indicated.

| | |
|---|---|
| Temperature: °C | degrees Centigrade |
| Length: cm | centimeters |
| Thickness: mil | 1 mil is equal to 25.4 μm |
| Weight: g | gram |
| mg | milligram |
| Volume: dL | deciliter |
| mL | milliliter |
| μL | microliter |
| L | liter |
| Concentration: | |
| mM | millimolar (millimoles per liter) |
| M | Molar (moles per liter) |
| % w/v | percent weight per deciliter |
| % v/v | percent volume per deciliter |
| Ions: | |
| Na⁺ | sodium ion |
| K⁺ | potassium ion |
| Li⁺ | lithium ion |
| Ca⁺⁺ | calcium ion |
| Mg⁺⁺ | magnesium ion |

Abbreviations for chemical components used are given below. The ionophore designations were assigned by the present inventors for convenience only. The name is usually based on the principal ion the ionophore was used to determined. However, the ionophores commonly respond, to varying lesser degrees, to other ions. Structures of the ionophores named can be found in EP—A—0 153 641.

7

Ionophores
Sodium Ionophore I                 1,1,1-*tris*-[1'-(2'-oxa-4'-oxo-5'-aza
                                    5'-methyl)-dodecanyl]propane

Sodium Ionophore II                N,N'-dibenzyl-N,N'-diphenyl-1,2-phenyl-
                                    enedioxydiacetamide

Sodium Ionophore III               6,7,9,10,18,19-hexahydro-17-*n*-butyl-di-
                                    benzo[b,k], [1,4,7,10,13] pentaoxacyclohexadecane-
                                    18-yl-oxyacetic acid

Potassium Ionophore I              2,3-naphthol-1,4,7,10,13-pentaoxacyclopentadeca-2-ene

Calcium Ionophore I                diethyl-N,N'-[(4R, 5R)-4,5-dimethyl-1,8-dioxo-
                                    3,6-dioxaocta-methylene]*bis*(12-methyl-
                                    aminododecanoate)

Hydrophobic Vehicle
NPOE                               2-nitrophenyl octyl ether

NPBE                               2-nitrophenyl butyl ether

Reporter Substance
7-decyl MEDPIN                     7-(*n*-decyl)-2-methyl-4-(3',5'-dichlorophen-
                                    4'-one)-indonaphthol

Miscellaneous
EDTA                               ethylenediamine tetraacetic acid

TRIS-Cl                            Tris(hydroxymethyl)-aminomethane hydro-
                                    chloride

EGTA                               ethyleneglycol-*bis*(aminoethyl)-
                                    tetraacetic acid (G. Fredrick Smith
                                    Chemical Co., Columbus, Ohio)

Triton® X-100                      polyethyleneglycol-*p*-isooctylphenyl ether
                                    (Sigma Chemical Co., St. Louis, MO.)

NaDDBS                             Sodium dodecyl benzene sulfonate

This invention will now be illustrated by the following examples.

8.1 Comparison Example: Multilayer potassium—Correlation of serum and whole blood measurements with the reference method

The potassium ion concentration determined from a two layer test device was compared to the determination of potassium with a flame photometer reference method. Blood from fifteen normal subjects was drawn and the potassium concentration determined with the device, using the reflectance measured from 155—180 seconds. Serum was prepared by centrifugation and assayed with the device in the same way. Aliquots were also assayed by the flame photometer reference method.

The reagent layer was prepared by emulsifying the hydrophobic vehicle, NPBE, containing 120 mM Potassium Ionophore I and 30 mM 7-decyl MEDPIN with a hydrophilic carrier and with the aid of butane-dioic acid, dodecyl-4,4'-[(1-methylethylidene)di-4,1-cyclohexanediyl] ester referred to herein as an oil former. The emulsion was coated and dried before the reflective layer was applied.

Reagent Layer:
    hydrophobic vehicle          8.3%
    gelatin                      8.3%
    oil former                   0.042%
    polystyrene sulphonate       0.02%
    Tris-Cl (pH 7.5)             0.2 M

Wet Thickness:
    375 µm

Reflective Layer:
gelatin 2.75%
titanium dioxide 19%

Wet Thickness:
82 µm

Percentages given are gm per 100 ml of the coating solution. Test devices were prepared with a transparent support and the reflectance measured through the support with a SERALYZER®. The data are shown below. The apparent potassium ion concentration (mM) for each data point is shown in parentheses. These apparent concentrations were calculated using the correlation equations for blood and serum.

| Sample # | Reference [K$^+$] concen. mM | Response from blood | Device, K/S serum |
|---|---|---|---|
| 1 | 4.40 | 1.484 (3.91) | 2.043 (4.00) |
| 2 | 3.80 | 1.399 (3.59) | 1.977 (3.71) |
| 3 | 3.85 | 1.536 (4.12) | 2.190 (4.65) |
| 4 | 3.80 | 1.525 (4.07) | 2.046 (4.02) |
| 5 | 3.65 | 1.444 (3.76) | 1.992 (3.78) |
| 6 | 3.55 | 1.380 (3.51) | 2.020 (3.90) |
| 7 | 3.55 | 1.451 (3.71) | 1.854 (3.17) |
| 8 | 3.85 | 1.585 (4.31) | 2.004 (3.83) |
| 9 | 3.90 | 1.433 (3.72) | 1.964 (3.65) |
| 10 | 3.85 | 1.303 (3.21) | 1.949 (3.59) |
| 11 | 4.25 | 1.456 (3.81) | 2.079 (4.16) |
| 12 | 4.00 | 1.391 (3.55) | 1.944 (3.57) |
| 13 | 4.35 | 1.731 (4.87) | 2.162 (4.53) |
| 14 | 4.65 | 1.734 (4.88) | 2.270 (5.01) |
| 15 | 4.25 | 1.679 (4.67) | 2.074 (4.14) |

For blood, the correlation equation is:

$$K/S = 0.258 \ [K^+] + 0.474; \quad [K^+] = \frac{K/S - 0.474}{0.258}$$

regression coefficient=0.653
standard error=0.10 K/S

For serum:

$$K/S = 0.226 \ [K^+] + 1.138; \quad [K^+] = \frac{K/S - 1.138}{0.226}$$

regression coefficient=0.713
standard error=0.076 K/S

The results show good correlation between the concentration of potassium ion in both blood and serum measured with the test device and that measured by flame photometry over the critical concentration range of 3 to 5 mM potassium.

8.3 Addition of sodium chloride to obviate hematocrit dependence of a whole blood potassium test

The volume fraction of blood volume occupied by red blood cells, the hematocrit, is normally 40 to 42%. Inside the red blood cell the normal concentration of potassium ion is approximately 120 mM, whereas that in the serum is approximately 4 mM. Hence a potassium determination on whole blood is sensitive to a very small degree of red blood cell destruction (hemolysis).

While the hematocrit is usually 40 to 42%, it can range from 30 to 70% in pathological states. It was found that the response of a single layer potassium test device was sensitive to the value of the hematocrit. This could lead to erroneous measured values of potassium ion concentration. A series of devices were prepared having a substantially common formula, but varying in the amount of sodium chloride (NaCl) added to the reagent or the reflective layers.

An emulsion of an oil phase dispersed in a continuous aqueous phase containing gelatin was prepared using a high shear emulsifier. The oil former used was the same as that used in Comparison Example 8.1. The composition of the oil phase was

| Potassium Ionophore I | 120 mM |
| 7-decyl MEDPIN | 30 mM |
| oil former | 0.6% |
| NPBE | remainder |

The final composition of the emulsion was

| Oil phase | 8.31% |
| Gelatin | 8.31% |
| Polystyrene sulfonic acid | 0.0207% |
| Tris-Cl buffer pH 7.5 | X |
| NaCl | (shown on table) |

Percentages given are weight per volume of the phase referred to. The concentration of Tris-Chloride used as the buffer, X, in device 1 was 0.2 M; the remaining devices were prepared with 0.166 M Tris-Cl buffer. The reagent layer was cast to a wet thickness of 175 μm on a polyester backing. The reflective layer contained

| titanium dioxide | 19% |
| gelatin | 2.75% |
| Tris-Cl buffer pH 7.5 | X |
| Triton X-100 | 0.03% |
| NaCl | (shown on table) |

The concentration of Tris-Cl buffer, X, used in device 1 was 0.1 M; the remaining devices were prepared with 0.062 M Tris-Cl buffer. The reflective layer was then spread on the dried reagent layer to a wet thickness of 82 μm.

| | | Concentration NaCl (M) | |
| Formulation | [Reagent] layer | [Reflective] layer | Overall* |
|---|---|---|---|
| 1 | 0 | 0 | 0 |
| 2 | 0.05 | 0.05 | 0.05 |
| 3 | 0.15 | 0.05 | 0.12 |
| 4 | 0.05 | .5 | 0.19 |
| 5 | 0.15 | .5 | 0.26 |

* The overall concentration of sodium chloride was calculated by adding the salt concentration in each layer from the equation:

$$\text{Overall} = \frac{[\text{reagent}] + [\text{reflective}]}{(\text{reagent} + \text{reflective layer}) \text{ thickness}}$$

10

wherein concentration in the reagent layer is found by multiplying the reagent layer thickness by the sodium chloride concentration in that layer and concentration in the reflective layer is found by multiplying the reflective layer thickness by the sodium chloride concentration in the reflecting layer.

Test devices were prepared and the potassium ion response measured as described previously. Samples of varying hematocrit were prepared. A high hematocrit sample (about 60%) was generated by gentle centrifugation and removal of some serum. A range of samples having the same potassium ion concentration but varying hematocrit was prepared by adding back different amounts of the autologous serum.

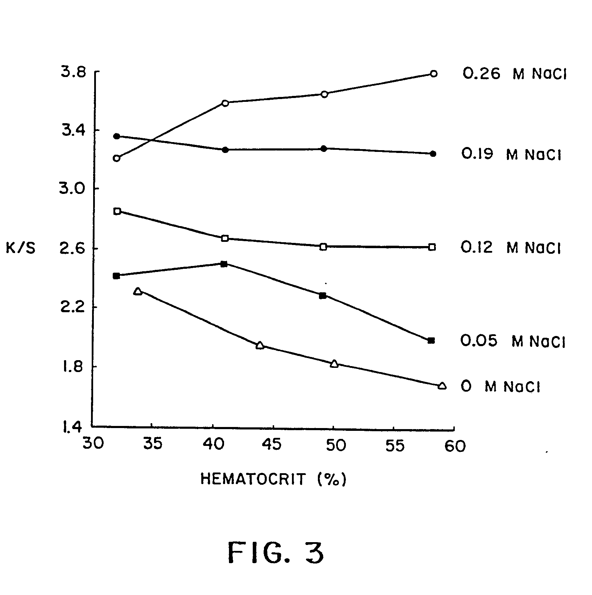

Assay of the samples with the five device formulations showed that with less than about 0.1 M sodium chloride, the potassium ion response decreases with increasing hematocrit, with too much sodium chloride the response increases. The addition of from about 0.1 to about 0.2 M overall sodium chloride provides a test device essentially independent of hematocrit. These results are shown in Figure 3. The overall molar concentration of sodium chloride in the test device formulation is marked for each response curve.

**Claims**

1. A multilayer test means for determining the presence of an ion in whole blood, the test means comprising:

a) a reagent layer consisting of

(i) a substantially nonpolar, nonporous carrier matrix incorporated with an ionophore capable of forming a complex with a specific ion to be determined and a neutral reporter substance having a dissociable proton, which proton dissociates upon interaction of the reporter with the complex of the ionophore and the ion to produce a detectable response; and

(ii) a hydrophilic carrier matrix incorporated with finely divided globules of a hydrophobic vehicle, the globules containing an ionophore forming a complex with a specific ion to be determined and a neutral reporter substance having a dissociable proton which proton dissociates upon interaction of the reporter with the complex of the ionophore and the ion to produce a detectable response;

b) a reflecting layer disposed on top of the reagent layer and in laminar relationship thereto, the reflecting layer including one or more materials which act as a background in order to facilitate the determination of a detectable response in the reagent layer; and optionally,

c) an opacifying layer in laminar relationship to the reflecting layer, the opacifying layer containing particles for imparting an opaque appearance to the layer, characterized in that said test means additionally comprises sodium chloride incorporated in one of the layers to impart hematocrit independence to the ion determination.

2. The multilayer test means of claim 1 in which the total concentration of sodium chloride added is between 0.1 and 0.2 molar.

3. The multilayer test means of claim 1 in which the ion determined is potassium ion.

4. The multilayer test means of claim 1 in which the ion determined is ultrafiltrable calcium ion.

5. The multilayer test means of claim 1 which additionally comprises a buffer in one of the layers.

**Patentansprüche**

1. Vielschichtige Testvorrichtung zum Bestimmen der Anwesenheit eines Ions in Gesamtblut, wobei die Testvorrichtung umfasst:

(a) eine Reagensschicht, bestehend aus

(i) einer im wesentlichen unpolaren, nicht-porösen Trägermatrix, inkorporiert mit einem Ionophor, der in der Lage ist, mit einem zu bestimmenden spezifischen Ion einen Komplex auszubilden, und einer neutralen Reportersubstanz, die ein dissoziierbares Proton hat, wobei das Proton aufgrund Wechselwirkung des Reporters mit dem Komplex aus dem Ionophor und dem Ion unter Ausbildung einer nachweisbaren Wirkung dissoziiert; und

(ii) einer hydrophilen Trägermatrix, inkorporiert mit feinverteilten Kügelchen eines hydrophoben Vehikels, wobei die Kügelchen einen Ionophor, der einen Komplex mit einem zu bestimmenden, spezifischen Ion bildet, enthält, und einer neutralen Reportersubstanz, die ein dissoziierbares Proton hat, wobei das Proton aufgrund der Wechselwirkung des Reporters mit dem Komplex aus dem Ionophor und dem Ion unter Erhalt einer nachweisbaren Wirkung dissoziiert;

(b) eine Reflektierschicht, die oben auf die Reagensschicht und in einem laminaren Verhältnis dazu angebracht ist, wobei die Reflektierschicht ein oder mehrere Materialien miteinschliesst, die als Hintergrund wirken, um die Bestimmung der nachweisbaren Wirkung in der Reagensschicht zu erleichtern; und gegebenenfalls

(c) eine opazifierende Schicht in einer laminaren Beziehung zur Reflektierschicht, wobei die opazifierende Schicht Partikel zum Vermitteln eines opaken Aussehens der Schicht enthält, dadurch gekennzeichnet, dass die Testvorrichtung zusätzlich Natriumchlorid inkorporiert in eine der Schichten umfasst, um der Ionenbestimmung Hämatokrit-Unabhängigkeit zu verleihen.

2. Vielschichtige Testvorrichtung gemäss Anspruch 1, in der die Gesamtkonzentration von zugegebenem Natriumchlorid zwischen 0,1 und 0,2 molar ist.

11

3. Vielschichtige Testvorrichtung gemäss Anspruch 1, in der das zu bestimmende Ion das Kaliumion ist.

4. Vielschichtige Testvorrichtung gemäss Anspruch 1, in der das zu bestimmende Ion ultrafiltrierbares Kalziumion ist.

5. Vielschichtige Testvorrichtung gemäss Anspruch 1, welche zusätzlich einen Puffer in einer der Schichten umfasst.

**Revendications**

1. Dispositif analytique multicouche pour la détection de la présence d'un ion dans du sang entier, ce dispositif analytique comprenant:

a) une couche réactive constituée

(i) d'une matrice de support non poreuse pratiquement non polaire renfermant un ionophore capable de former un complexe avec un ion spécifique devant être détecté et une substance indicatrice neutre portant un proton dissociable, lequel proton se dissocie par interaction de la substance indicatrice avec le complexe de l'ionophore et de l'ion en engendrant une réponse détectable; et

(ii) d'une matrice de support hydrophile renfermant des globules finement divisés d'un véhicule hydrophobe, les globules contenant un ionophore formant un complexe avec un ion spécifique devant être dosé et une substance indicatrice neutre portant un proton dissociable, lequel se dissocie par interaction de la substance indicatrice avec le complexe de l'ionophore et de l'ion en engendrant une réponse détectable;

b) une couche réfléchissante disposée par-dessus la couche réactive et en relation laminaire avec elle, la couche réfléchissante comprenant une ou plusieurs substances qui agissent en arrière-plan de manière à faciliter l'appréciation d'une réponse détectable dans la couche réactive; et à titre facultatif

c) une couche opacifiante en relation laminaire avec la couche réfléchissante, la couche opacifiante contenant des particules lui conférant un aspect opaque, caractérisé en ce qu'il comprend en outre du chlorure de sodium incorporé dans l'une des couches afin de créer une indépendance à l'égard de l'hématocrite pour le dosage des ions.

2. Dispositif analytique multicouche suivant la revendication 1, dans lequel la concentration totale en chlorure de sodium ajoutée va de 0,1 à 0,2 M.

3. Dispositif analytique multicouche suivant la revendication 1, dans lequel l'ion dosé est l'ion potassium.

4. Dispositif analytique multicouche suivant la revendication 1, dans lequel l'ion dosé est l'on calcium ultrafiltrable.

5. Dispositif analytique multicouche suivant la revendication 1, qui comprend en outre un tampon dans l'une des couches.

FIG. Ia

FIG. Ib

FIG. 2a

FIG. 2b

FIG. 3